# EUROPEAN PATENT APPLICATION

(11) **EP 4 556 472 A1**
(43) Date of publication of application: **21.05.2025**
(21) Application number: 23839021.5
(22) Date of filing: 13.07.2023
(51) Int. Cl.: C07D 409/14, C07D 405/04, A61K 31/4436, A61K 31/4427, A61P 1/00, A61P 1/16

(54) **USE OF PYRIDONE DERIVATIVE**

(30) Priority: 14.07.2022 CN 202210834767
(71) Applicant: GUANGZHOU JOYO PHARMATECH CO., LTD, Guangzhou, Guangdong 510663 (CN)
(72) Inventor: LI, Yongguo, Guangzhou, Guangdong 510663 (CN); CHEN, Xiaoning, Shanghai 201203 (CN); SONG, Haoliang, Shanghai 201203 (CN); LI, Hongjiao, Hangzhou, Zhejiang 310052 (CN)
(74) Representative: Secerna LLP
(86) International application number: PCT/CN2023/107252
(87) International publication number: WO 2024/012531

(57) **Abstract**

A use of a pyridone derivative. A use of a substance A in preparation of a drug, wherein the substance A is a compound as shown in formula I or a pharmaceutically acceptable salt thereof; and the drug is a drug for treating and/or preventing intestinal fibrosis or non-alcoholic liver fibrosis. The substance A or the drug containing the substance A can achieve a good drug effect on intestinal fibrosis or non-alcoholic liver fibrosis at a small dosage.

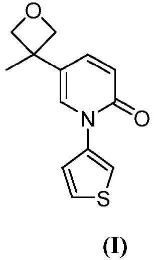

## Description

The present application claims the right of the priority of Chinese patent application No. 2022108347679 filed on July 14, 2022. The contents of the above Chinese patent application are incorporated herein by reference in its entirety.

### TECHNICAL FIELD

The present disclosure relates to the technical field of biomedicine, and specifically relates to a use of a pyridone derivative in the manufacture of a medicament for treating fibrosis.

### BACKGROUND

Fibrosis is a systemic disease that can affect any organ, most commonly the heart, lungs, kidneys, liver, and skin. It is a result of dysregulated tissue repair responses in various types of tissue damage, especially in chronic inflammatory diseases. Fibrosis in different fibrous proliferative diseases has their own unique mechanisms. It has been reported that the number of deaths caused by fibrosis accounts for 45% of the total deaths in the industrialized world.

The primary drivers of intestinal fibrosis are soluble molecules (e.g., cytokines and growth factors), G-protein-coupled receptors, epithelial-mesenchymal transition, endothelialmesenchymal transition, intestinal microbiota, *etc.* Although immunosuppressants and antiinflammatory biologics can alleviate inflammation and alleviate related clinical symptoms, they do not have direct anti-fibrotic effects and cannot reduce the intestinal fibrosis that has already occurred or prevent the subsequent fibrosis process. Therefore, there are currently no approved therapeutic drugs for intestinal fibrosis, and the treatment options for intestinal stenosis caused by intestinal fibrosis are still mainly surgery and endoscopic treatments (D'Alessio S et al., Nat Rev Gastroenterol Hepatol. 2022 Mar; 19(3): 169-184.; Hu Fan et al., Chinese Journal of Medical Frontiers (Electronic Edition), 2021, Vol. 13, Issue 7: 14-21).

Non-alcoholic liver fibrosis refers to abnormal proliferation of connective tissue in the liver caused by factors other than alcohol.

For a long time, fibrosis was considered irreversible; however, preclinical models and clinical trials have demonstrated that it is a highly dynamic process. Pirfenidone was initially approved for idiopathic pulmonary fibrosis (IPF), and subsequent studies have shown its therapeutic effect on fibrosis diseases in other fibrosis models. However, pirfenidone has poor efficacy and strong side effects, so it is urgent to find new anti-fibrotic drugs.

### CONTENT OF THE PRESENT INVENTION

The technical problem to be solved by the present disclosure is that the dose of the existing drugs for treating intestinal fibrosis or non-alcoholic liver fibrosis is large. The present disclosure provides a use of a pyridone derivative, which can treat and/or prevent intestinal fibrosis and non-alcoholic liver fibrosis with less use of such compounds.

The present disclosure provides a use of substance A in the manufacture of a medicament, wherein the substance A is a compound of formula I or a pharmaceutically acceptable salt thereof; the medicament is a medicament for treating and/or preventing intestinal fibrosis or non-alcoholic liver fibrosis;

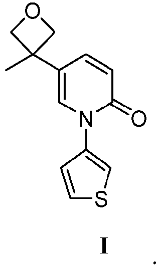

In the use, the intestinal fibrosis is preferably intestinal fibrosis caused by inflammatory bowel disease.

In the use, the intestinal fibrosis is more preferably intestinal fibrosis in Crohn's disease.

In the use, the medicament may be administered at a frequency of once a day, twice a day, or three times a day, preferably twice a day.

In the use, the unit dose of the medicament may be determined according to the effective dose of the subject/patient; the unit dose of the substance A may be 25 to 900 mg, preferably 50 to 600 mg, more preferably 200 to 400 mg.

In the use, each time the medicament is administered, the single dose of the medicament may be determined according to the actual therapeutic effect of a subject/patient; preferably, the single dose of the medicament is the mass ratio of the substance A to a subject/patient; the mass ratio may be 3 to 40 mg/kg, preferably 3 mg/kg, 6 mg/kg, 10 mg/kg, 15 mg/kg, 30 mg/kg, or 40 mg/kg.

In the use, when the medicament is a medicament for treating and/or preventing intestinal fibrosis, the single dose of the medicament may be the mass ratio of the substance A to a subject/patient; the mass ratio may be 3 to 40 mg/kg, preferably 3 mg/kg, 6 mg/kg, 10 mg/kg, 15 mg/kg, 30 mg/kg, or 40 mg/kg.

In the use, when the medicament is a medicament for treating and/or preventing non-alcoholic liver fibrosis, the single dose of the medicament may be the mass ratio of the substance A to a subject/patient; the mass ratio may be 3 to 40 mg/kg, preferably 3 mg/kg, 6 mg/kg, 10 mg/kg, 15 mg/kg, 30 mg/kg, or 40 mg/kg.

In the use, each time the medicament is administered, the daily dose of the medicament may be determined according to the actual therapeutic effect of a subject/patient; preferably, the daily dose of the medicament is the mass ratio of the substance A to a subject/patient; the mass ratio may be 3 to 80 mg/kg, preferably 3 mg/kg, 6 mg/kg, 12 mg/kg, 15 mg/kg, 30 mg/kg, 40 mg/kg, 60 mg/kg, or 80 mg/kg.

In the use, when the medicament is a medicament for treating and/or preventing intestinal fibrosis, the daily dose of the medicament may be the mass ratio of the substance A to a subject/patient; the mass ratio may be 3 to 80 mg/kg, preferably 3 mg/kg, 6 mg/kg, 12 mg/kg, 15 mg/kg, 30 mg/kg, 40 mg/kg, 60 mg/kg, or 80 mg/kg.

In the use, when the medicament is a medicament for treating and/or preventing non-alcoholic liver fibrosis, the daily dose of the medicament may be the mass ratio of the substance A to a subject/patient; the mass ratio is 3 to 80 mg/kg, preferably 3 mg/kg, 6 mg/kg, 12 mg/kg, 15 mg/kg, 30 mg/kg, 40 mg/kg, 60 mg/kg, or 80 mg/kg.

In the use, the compound of formula I may be present in the medicament at a concentration of 0.3 to 3 mg/mL, preferably 0.3 mg/mL, 0.6 mg/mL, 1 mg/mL, 1.5 mg/mL, or 3 mg/mL.

In the use, the medicament may comprise the substance A and a pharmaceutically acceptable carrier.

In the use, the pharmaceutically acceptable carrier may be a conventional carrier in the art.

In the use, the medicament may be administered via the gastrointestinal tract, preferably orally.

In the use, the dosage form of the medicament may be a conventional dosage form in the art, and the dosage form of the medicament may be a solid formulation.

In the use, the mass fraction of the compound of formula I in the medicament is 3% to 50%, preferably 10 to 20%.

The present disclosure further provides a pharmaceutical composition comprising substance A and a pharmaceutically acceptable carrier; the substance A is a compound of formula I or a pharmaceutically acceptable salt thereof; 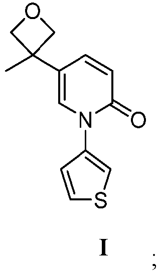 in the pharmaceutical composition, the compound of formula I has a mass fraction of 3% to 50%.

In the pharmaceutical composition, the compound of formula I has a mass fraction of 10% to 20%.

The present disclosure further provides a use of the pharmaceutical composition in the manufacture of a medicament for treating and/or preventing intestinal fibrosis or non-alcoholic liver fibrosis.

In the use, the intestinal fibrosis is preferably intestinal fibrosis caused by inflammatory bowel disease, more preferably intestinal fibrosis in Crohn's disease.

The present disclosure further provides a method for treating and/or preventing intestinal fibrosis or non-alcoholic liver fibrosis, wherein the method comprises administering a therapeutically effective amount of substance A or a medicament to a subject/patient; the medicament comprises the substance A and a pharmaceutically acceptable carrier; the substance A is a compound of formula I or a pharmaceutically acceptable salt thereof; 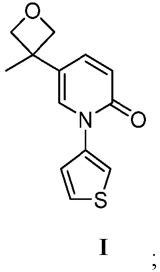 in the method, the pharmaceutically acceptable carrier is a conventional carrier in the art.

In the method, the intestinal fibrosis is preferably intestinal fibrosis caused by inflammatory bowel disease, more preferably intestinal fibrosis in Crohn's disease.

In the method, the medicament may be administered at a frequency of once a day, twice a day, or three times a day, preferably twice a day.

In the method, the unit dose of the medicament may be determined according to the effective dose of the subject/patient; preferably, the unit dose of the substance A may be 25 to 900 mg, preferably 50 to 600 mg, more preferably 200 to 400 mg.

In the method, each time the medicament is administered, the single dose of the medicament may be determined according to the actual therapeutic effect of a subject/patient; preferably, the single dose of the medicament may be the mass ratio of the substance A to a subject/patient; the mass ratio may be 3 to 40 mg/kg, preferably 3 mg/kg, 6 mg/kg, 10 mg/kg, 15 mg/kg, 30 mg/kg, or 40 mg/kg.

In the method, when the medicament is a medicament for treating and/or preventing intestinal fibrosis, the single dose of the medicament may be the mass ratio of the substance A to a subject/patient; the mass ratio may be 3 to 40 mg/kg, preferably 3 mg/kg, 6 mg/kg, 10 mg/kg, 15 mg/kg, 30 mg/kg, or 40 mg/kg.

In the method, when the medicament is a medicament for treating and/or preventing non-alcoholic liver fibrosis, the single dose of the medicament may be the mass ratio of the substance A to a subject/patient; the mass ratio may be 3 to 40 mg/kg, preferably 3 mg/kg, 6 mg/kg, 10 mg/kg, 15 mg/kg, 30 mg/kg, or 40 mg/kg.

In the method, each time the medicament is administered, the daily dose of the medicament may be determined according to the actual therapeutic effect of a subject/patient; preferably, the daily dose of the medicament may be the mass ratio of the substance A to a subject/patient; the mass ratio may be 3 to 80 mg/kg, preferably 3 mg/kg, 6 mg/kg, 12 mg/kg, 15 mg/kg, 30 mg/kg, 40 mg/kg, 60 mg/kg, or 80 mg/kg.

In the method, when the medicament is a medicament for treating and/or preventing intestinal fibrosis, the daily dose of the medicament may be the mass ratio of the substance A to a subject/patient; the mass ratio may be 3 to 80 mg/kg, preferably 3 mg/kg, 6 mg/kg, 12 mg/kg, 15 mg/kg, 30 mg/kg, 40 mg/kg, 60 mg/kg, or 80 mg/kg.

In the method, when the medicament is a medicament for treating and/or preventing non-alcoholic liver fibrosis, the daily dose of the medicament may be the mass ratio of the substance A to a subject/patient; the mass ratio may be 3 to 80 mg/kg, preferably 3 mg/kg, 6 mg/kg, 12 mg/kg, 15 mg/kg, 30 mg/kg, 40 mg/kg, 60 mg/kg, or 80 mg/kg.

In the method, the compound of formula I may be present in the medicament at a concentration of 0.3 to 3 mg/mL, preferably 0.3 mg/mL, 0.6 mg/mL, 1 mg/mL, 1.5 mg/mL, or 3 mg/mL.

In the method, the medicament may be administered via the gastrointestinal tract, preferably orally.

In the method, the dosage form of the medicament may be a conventional dosage form in the art, and the dosage form of the medicament may be a solid formulation.

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable carrier" refers to any formulation or carrier medium that can deliver an effective amount of the active substance of the present disclosure, does not interfere with the biological activity of the active substance, and has no toxicity or side effects on the host or patient. Representative carriers include water, oils, vegetables, minerals, paste bases, lotion bases, ointment bases, *etc.* These bases include suspending agents, viscosity-increasing agents, transdermal enhancers, *etc.* Their formulations are well known to those skilled in the field of cosmetics or topical medication. For additional information on carriers, one can refer to Remington: The Science and Practice of Pharmacy, 21st Ed., Lippincott, Williams & Wilkins (2005), the contents of which are incorporated herein by reference.

The term "treat/treatment" refers to therapeutic therapy. When referring to a specific condition, "treat/treatment" refers to: (1) alleviating one or more biological manifestations of a disease or condition; (2) interfering with (a) one or more points in the biological cascade leading to or causing the condition or (b) one or more biological manifestations of the condition; (3) improving one or more symptoms, effects, or side effects associated with the condition, or one or more symptoms, effects, or side effects associated with the condition or its treatment, or (4) slowing the progression of one or more biological manifestations of the disease or condition.

The term "prevent/prevention" refers to a reduction in the risk of acquiring or developing a disease or disorder.

The term "therapeutically effective amount" refers to an amount of a compound that, when administered to a patient, is sufficient to effectively treat the disease or condition described herein. The term "therapeutically effective amount" will vary based on the compound, the disease and its severity, as well as the age of the patient to be treated, but can be adjusted as needed by those skilled in the art.

The term "patient" refers to any animal that will receive, or has already received, administration of the compound according to examples of the present disclosure, with mammals being preferred and humans being most preferred. The term "mammal" includes any mammal. Examples of mammals include, but are not limited to, cattle, horses, sheep, pigs, cats, dogs, mice, rats, rabbits, guinea pigs, monkeys, humans, *etc.,* with humans being most preferred.

On the basis of conforming to the common sense in this field, the above preferred conditions can be arbitrarily combined to obtain the preferred examples of the present disclosure.

The reagents and raw materials used in the present disclosure are all commercially available.

The positive progressive effects of the present disclosure are that:

The compound of formula I of the present disclosure can achieve good efficacy in treating and/or preventing intestinal fibrosis and non-alcoholic liver fibrosis with a relatively low dose. Further, the compound of formula I of the present disclosure is effective at a dose of 3.0 mg/kg for treating and/or preventing intestinal fibrosis and non-alcoholic liver fibrosis.

### BRIEF DESCRIPTION OF THE DRAWINGS

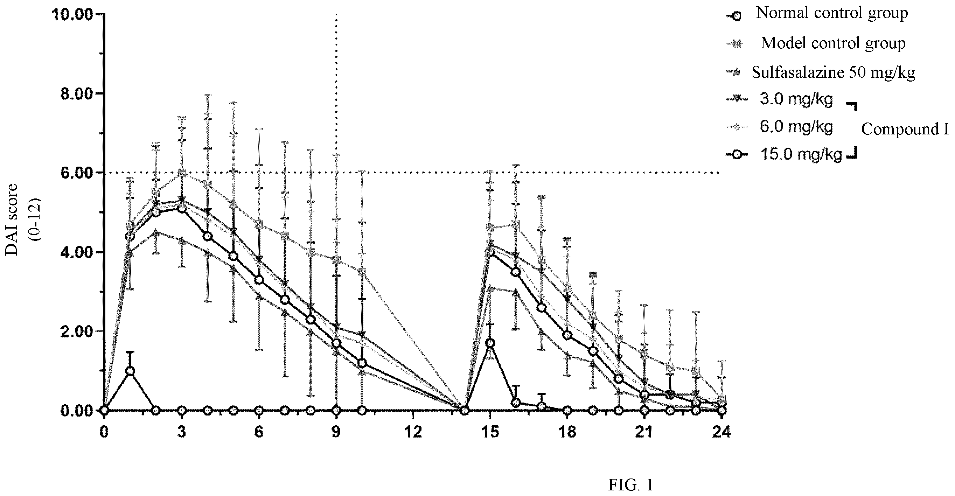

FIG. 1 shows the DAI scoring results of animals in each experimental group during the experimental period in Example 1; all data are statistically analyzed using mean ± standard deviation (SD);

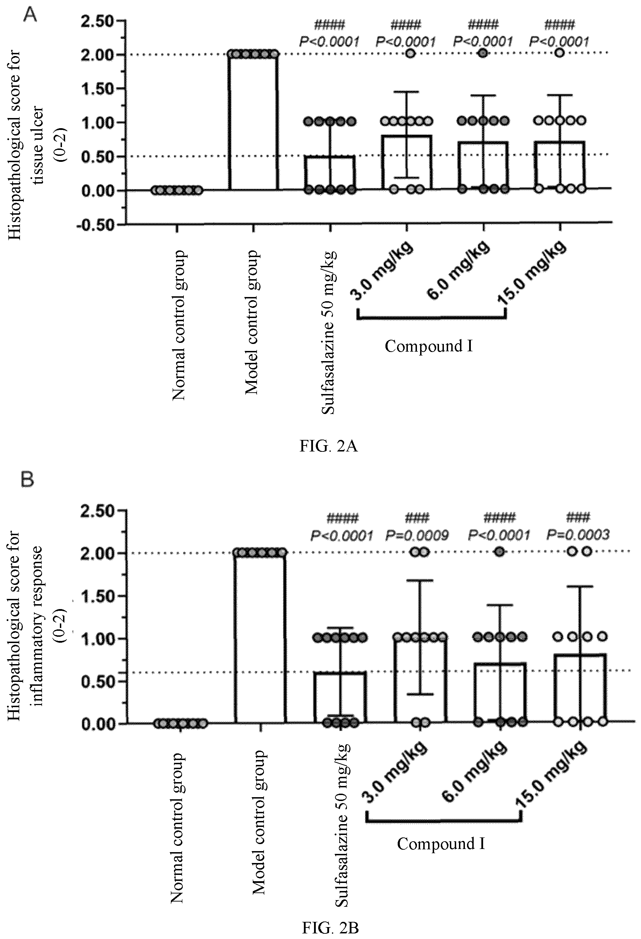

FIG. 2A shows the histopathological diagnosis scoring results of ulcers in the middle segment colon tissue of animals in each experimental group in Example 1;
FIG. 2B shows the histopathological scoring results of the inflammatory response of the middle segment colon tissue of animals in each experimental group in Example 1; all data in FIG. 2 are statistically analyzed using mean ± standard deviation (SD), #P < 0.05, ##P < 0.01, ###P < 0.001, ####P < 0.0001 represent the statistical analysis results of animals in different treatment groups and the model control group;

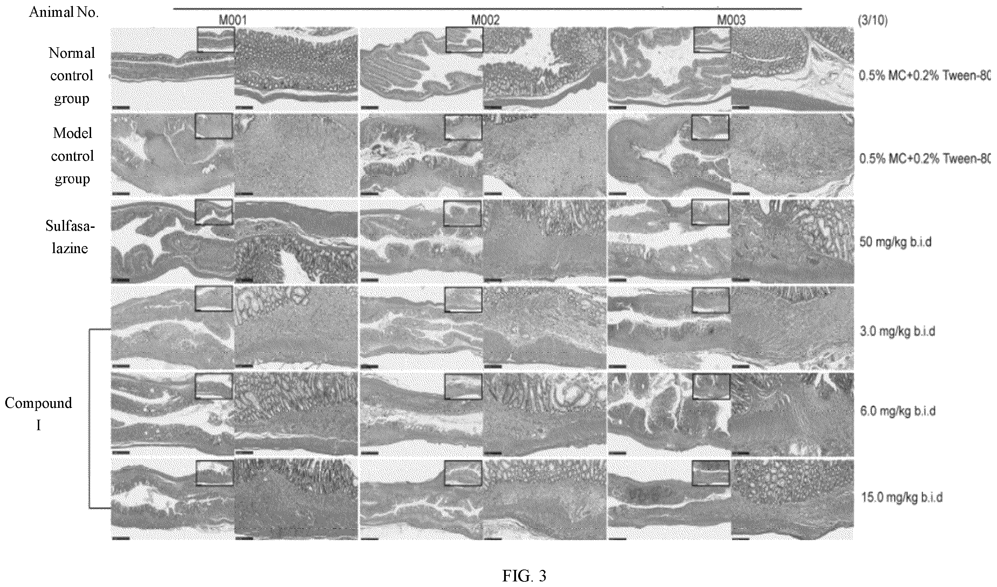

FIG. 3 shows representative images of ulcer lesions on colon tissues of animals in each experimental group in Example 1; representative histopathological images of the middle segment colon of animals in each experimental group are diagnosed and analyzed for histopathology under a light microscope at 100× and 200× magnification, respectively; three animals per experiment are selected for the display of results, with image scales of 1 mm, 250 µm, and 100 µm, respectively;

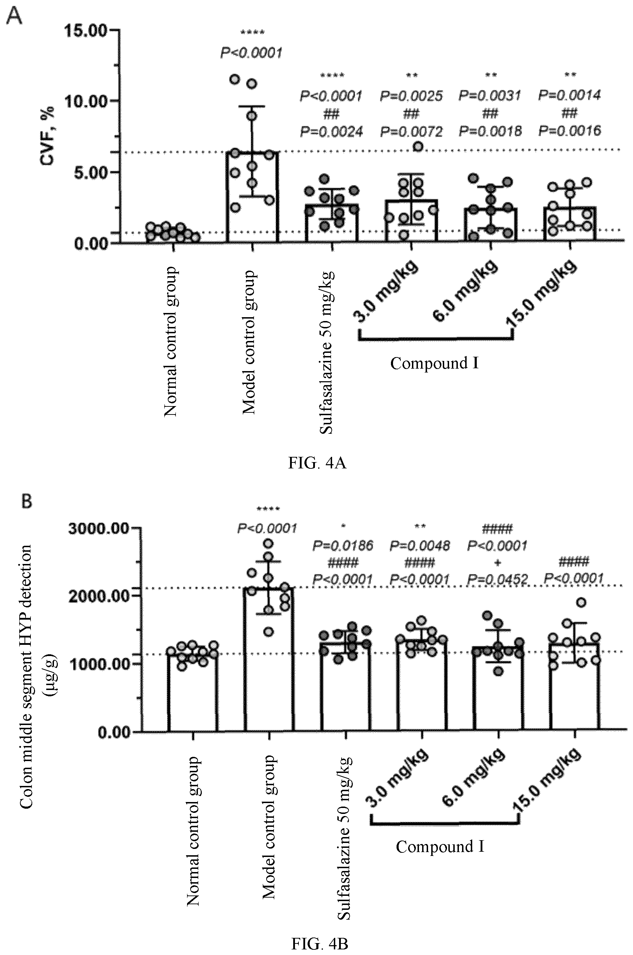

FIG. 4A shows the CVF% of colon tissue of animals in each experimental group in Example 1;
FIG. 4B shows the HYP detection results of the middle segment colon of animals in each experimental group in Example 1; all data in FIG. 4 are statistically analyzed using mean ± standard deviation (SD), *P < 0.05, **P < 0.01, ***P < 0.001, ****P < 0.0001 represent the statistical analysis results of different experimental groups and normal control group, and #P < 0.05, ##P < 0.01, ###P < 0.001, ####P < 0.0001 represent the statistical analysis results of animals in different treatment groups and the model control group;

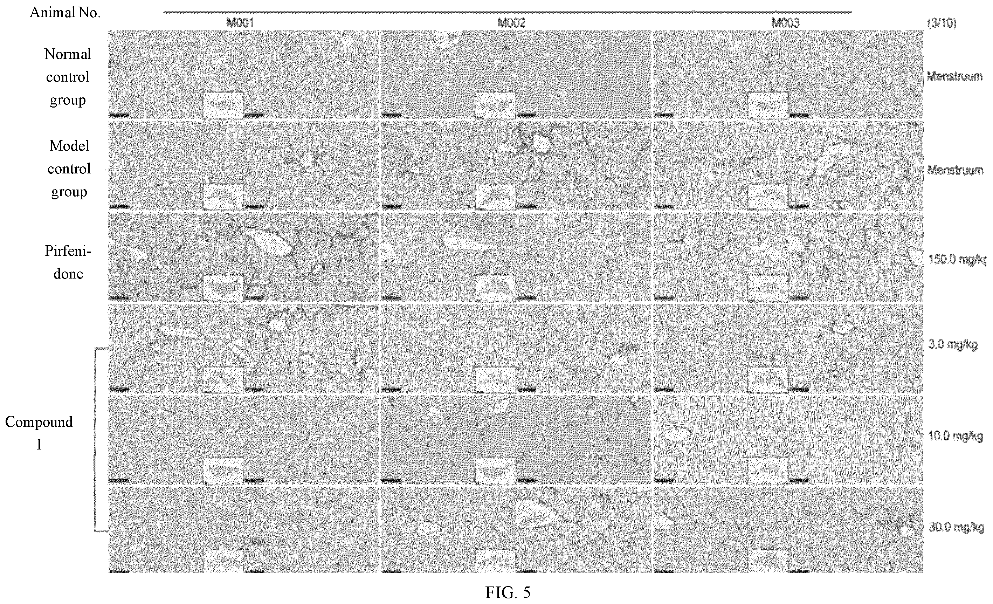

FIG. 5 shows the results of Sirius Red staining and diagnosis of animal livers in each experimental group during the experimental period in Example 2; the results of Sirius Red staining of animal liver tissue in each experimental group are diagnosed and analyzed for histopathology under a light microscope at 10×, 100×, and 200× magnification, respectively; three animals per experiment are selected for the display of results, with image scales of 1 mm, 250 µm, and 500 µm, respectively;

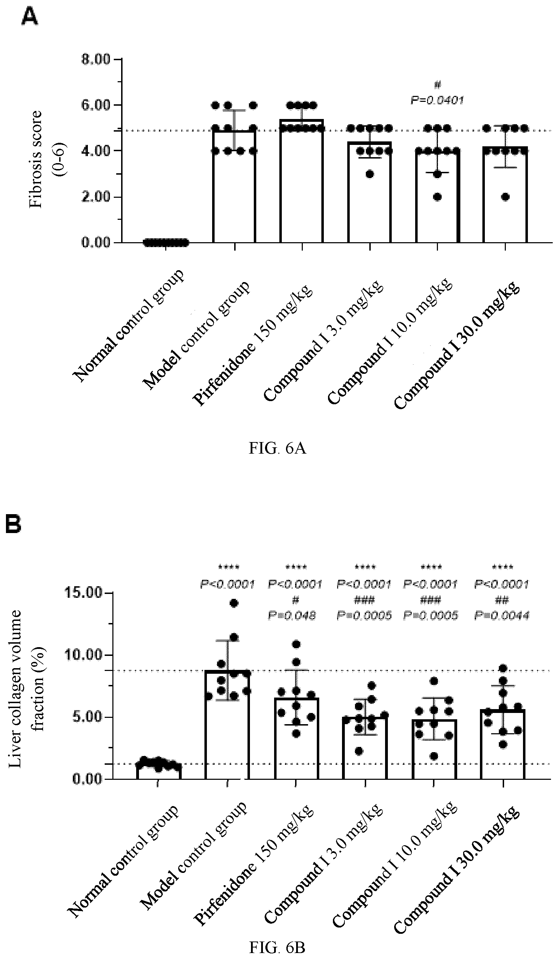

FIG. 6A shows the liver fibrosis scoring results of animals in each experimental group in Example 2;
FIG. 6B shows the calculation results of liver CVF% of animals in each experimental group in Example 2;

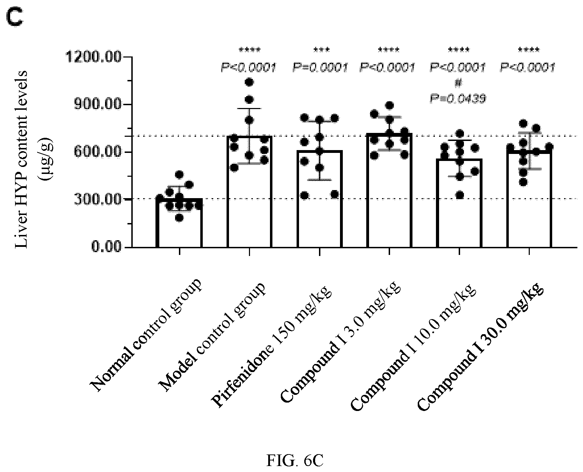

FIG. 6C shows the HYP detection results of animal livers in each experimental group in Example 2; all data are statistically analyzed using mean ± standard deviation (SD), *P < 0.05, **P < 0.01, ***P < 0.001, ****P < 0.0001 represent the statistical analysis results of different experimental groups and normal control group, and #P < 0.05, ##P < 0.01, ###P < 0.001, ####P < 0.0001 represent the statistical analysis results of animals in different treatment groups and the model control group.

### DETAILED DESCRIPTION OF THE EMBODIMENT

The present disclosure will be further illustrated by way of examples below, but it is not limited to the scope of these examples. The experimental methods for which the specific conditions are not specified in the following examples are selected according to the conventional methods and conditions, or according to the commodity instructions.

### Example 1: Pharmacodynamic experiment of TNBS-induced rat models of chronic colitis with intestinal fibrosis

1. Experimental objective
To evaluate the therapeutic effect of test compound I on TNBS-induced SD rat models of chronic colitis with intestinal fibrosis.
2. Experimental methods
1) Animals: SD rats, specific pathogen-free (SPF) grade, male, aged 7 to 8 weeks at the time of grouping, with 10 rats per group. The animals were supplied by Beijing HFK Bioscience Co., Ltd.
2) Reagents, positive drugs, and test drugs
A. Reagents

**Table 1. Sources of reagents**

| Name | Supplier |
|---|---|
| TNBS (2,4,6-trinitrobenzenesulfonic acid) | Sigma-Aldrich |
| 0.9% sodium chloride injection | Sichuan Kelun Pharmaceutical Co., Ltd. |
| Hydroxyproline detection kit | Nanjing Jiancheng Bioengineering Institute |

B. Positive drugs: sulfasalazine colon-soluble capsules, supplied by Guangdong Qiangji Pharmaceutical Co., Ltd.
C. Test drug: compound I, supplied by Guangzhou JOYO Pharmatech Co., Ltd.
3) Modeling method
TNBS two-enema method: After the adaptation period inspection and observation were qualified, experimental animals were screened by body weight and randomly divided into six experimental groups based on body weight. The groups included a normal control group, a model control group, a positive drug treatment group, a test compound low dose treatment group, a test compound medium dose treatment group, and a test compound high dose treatment group, with 10 rats in each group. On the day of grouping, animals in the normal control group were administered 50% ethanol solution via enema, while animals in the other groups were administered TNBS ethanol solution via enema. The dose of TNBS was 30 mg per animal, with the induction of the model performed twice at a frequency of once every two weeks. The experimental period lasted for 4 weeks, during which all animals were given normal access to water and food.
4) Grouping and administration

**Table 2. Grouping and administration info**

| Group | Number of animals | Drug | Route of administration | Dose | Concentration (mg/mL) | Frequency and period of administration |
|---|---|---|---|---|---|---|
| Normal control group | 10 | Test compound vehicle^{a} | Oral gavage | - | - | Twice a day for four consecutive weeks |
| Model control group | 10 | Test compound vehicle^{a} | Oral gavage | - | - | |
| Positive drug treatment group | 10 | Sulfasalazine^{b} | Oral gavage | 50 mg/kg | 5 | |
| Test compound low dose treatment group | 10 | Compound I^{c} | Oral gavage | 3 mg/kg | 0.3 | |
| Test compound medium dose treatment group | 10 | Compound I^{c} | Oral gavage | 6 mg/kg | 0.6 | |
| Test compound high dose treatment group | 10 | Compound I^{c} | Oral gavage | 15 mg/kg | 1.5 | |

| | | | | | | |
|---|---|---|---|---|---|---|
| Note: a: The test compound vehicle was an aqueous solution of 0.5% (w/v, percentage of methylcellulose mass in the solution volume) methylcellulose and 0.2% (v/v, percentage of Tween 80 volume in the solution volume) Tween 80. b: A normal saline solution of 0.5% CMC-Na (sodium carboxymethylcellulose) was used as the vehicle and prepared to the required concentration. c: An aqueous solution of 0.5% (w/v) methylcellulose and 0.2% (v/v) Tween 80 was used as the vehicle and prepared to the required concentration. | | | | | | |

5) Determination of clinical indications
The Disease Activity Index (DAI) scoring was performed on each group of experimental animals based on indicators such as animal body weight, stool morphology and characteristics, and occult blood or hematochezia. Scoring was performed after model induction, once a day for 10 consecutive days, for a total of 2 rounds. The DAI scoring criteria are shown in Table 3;

**Table 3. DAI scoring criteria**

| Disease Activity Index (DAI) scoring criteria | | | |
|---|---|---|---|
| Score | Percentage of body weight loss | Stool consistency | Occult blood level in stool |
| 0 | Normal | Normal | Negative |
| 1 | 1-5% | Semiformed stool | Weak positive occult blood |
| 2 | 5-10% | Mushy stool | Positive occult blood |
| 3 | 10-20% | Loose stool | Weak positive hematochezia |
| 4 | >20% | Watery stool | Strong positive hematochezia |

| | | | |
|---|---|---|---|
| Note: The DAI score was performed based on the percentage of body weight loss, stool morphology, and progression of blood in the stool. Statistical analysis was conducted comprehensively using these three scoring indicators. | | | |

6) Endpoint sample collection, histopathological analysis, and hydroxyproline content analysis
A. After euthanizing the animals with pentobarbital sodium anesthesia, the colon, *i.e.,* the segment from the end of cecum to the anus, was excised. The intestinal segment was flushed with normal saline, and the entire colon was evenly divided into upper, middle, and lower segments. The middle segment of the colon was further divided, with part of the tissue quickly frozen in liquid nitrogen and stored in a -80°C refrigerator for measuring the total hydroxyproline levels in the middle segment samples of the colon of animals in each group. The remaining middle segment tissue was fixed in 10% formaldehyde, embedded in paraffin, and sectioned into 3 µm-thick slices. These slices were then deparaffinized in xylene, rehydrated through a graded ethanol series, subjected to H&E staining and Masson staining, dehydrated, cleared, sealed, and observed under a microscope to capture histopathological changes. Routine tissue freezing was performed.
B. Histopathological analysis

**Table 4. Scoring criteria for histological damage in colon**

| Histological damage manifestation | Pathological description | Score |
|---|---|---|
| Tissue ulcer | None | 0 |
| | Diameter of ulcer lesion < 3 mm | 1 |
| | Diameter of ulcer lesion > 3 mm | 2 |
| Inflammatory response | None | 0 |
| | Mild | 1 |
| | Severe | 2 |
| Submucosal and intestinal wall fibrosis | Calculated as a collagen volume fraction based on Masson staining results | CVF %^{a} |

| | | |
|---|---|---|
| Note: a: Based on the Masson staining results, the area of collagen staining-positive regions and the total area of samples from the middle segment samples of the colon (submucosa and muscularis) of animals in each experimental group were statistically analyzed using Image J to calculate the collagen volume fraction (CVF%) of each sample. | | |

C. Detection of hydroxyproline levels in the colon

The middle segment samples of the colon were processed according to the instructions, using the hydroxyproline detection kit. The total hydroxyproline levels in the middle segment samples of the colon of animals in each group were measured.

### 4. Experimental results

### 1) Clinical scoring

**Table 5. AUC (Area Under the Curve) of DAI scores of animals in each experimental group during the experimental period**

| Group | AUC_{(0-10d)} (D0-D10) | Change rate of AUC_{(0-10d)} (%) | AUC_{(14-24d)} (D14-D24) | Change rate of AUC_{(14-24d)} (%) |
|---|---|---|---|---|
| Normal control group | 1.00 ± 0.33 | - | 2.00 ± 0.51 | - |
| Model control group | 45.75 ± 4.72 | - | 24.05 ± 2.93 | - |
| Positive drug treatment group | 29.80 ± 2.79^{##} | -34.86 | 11.70 ± 1.69^{##} | -51.35 |
| Compound I low dose group | 37.15 ± 4.93^{#} | -18.80 | 19.30 ± 2.79^{##} | -19.75 |
| Compound I medium dose group | 36.05 ± 4.87^{#} | -21.20 | 17.25 ± 3.16^{##} | -28.27 |
| Compound I high dose group | 33.50 ± 3.97^{##} | -26.78 | 15.40 ± 3.50^{##} | -35.97 |

Results: After the first TNBS induction (D0-D10), the DAI scoring results indicated that animals in the model control group exhibited significant characteristics of colitis following TNBS intervention. Specific characteristics were manifested as obvious inhibition of body weight gain, reduced activity levels of animals, mushy to loose stools, and inflammation with swelling around the anus. The calculated AUC (0-10d) value for the model control group was 45.75 ± 4.72, which showed a statistically significant difference (P < 0.05) compared to the normal control group animals in the same period.

Test compound I and the positive drug, sulfasalazine, were administered to the respective groups of mice following the established treatment regimen. Based on the AUC (0-10d) data for each group, both compound I and sulfasalazine demonstrated a certain degree of improvement in the disease process of chronic colitis in the model animals. Compared to the positive drug control group, the calculated AUC (0-10d) values for each treatment group were 37.15 ± 4.93, 36.05 ± 4.87, 33.50 ± 3.97, and 29.80 ± 2.79, respectively, which decreased by 18.80%, 21.20%, 26.78%, and 34.86%, respectively, relative to the model control group in the same period. The test compound I was effective at a low dose, being 3.0 mg/kg.

After the second TNBS induction (D14-D24), the DAI scores significantly increased, and animals in the model control group exhibited obvious chronic colitis disease characteristics, mainly manifesting as two aspects: mushy and loose stools along with pronounced inflammation and swelling around the anus. The degree of weight loss was less severe than during the first induction. The DAI scores for the model control group showed statistically significant differences (P < 0.05) compared to the normal control group in the same period. Subsequently, the scores gradually decreased, returning to normal on Day 24, with no significant differences observed compared to the normal control group. The calculated AUC (14-24d) value for the model control group was 24.05 ± 2.93.

Test compound I and the positive drug, sulfasalazine, still exhibited a certain degree of improvement in the chronic colitis of the model animals. Compared to the model control group animals in the same period, the DAI scores of all treatment group animals showed some degrees of reduction. The calculated AUC (14-24d) values of DAI scores during the scoring period were 19.30 ± 2.79, 17.25 ± 3.16, 15.40 ± 3.50, and 11.70 ± 1.69, respectively, which decreased by 19.75%, 28.27%, 35.97%, and 51.35%, respectively, relative to the model control group in the same period.

The DAI scoring results of the animals in each experimental group during the experimental period are shown in FIG. 1.

2) Histopathology of colon tissue

**Table 6. Histopathological scoring results for colon tissue from animals in each experimental group**

| Group | Number of animals (pieces) | Tissue ulcer (points) | Inflammatory response (points) |
|---|---|---|---|
| Normal control group | 10 | 0 | 0 |
| Model control group | 10 | 2.00 ± 0.00 | 2.00 ± 0.00 |
| Positive drug treatment group | 10 | 0.50 ± 0.53^{####} | 0.60 ± 0.52^{####} |
| Compound I low dose group | 10 | 0.80 ± 0.63^{####} | 1.00 ± 0.67^{###} |
| Compound I medium dose group | 10 | 0.70 ± 0.67^{####} | 0.70 ± 0.67^{####} |
| Compound I high dose group | 10 | 0.70 ± 0.67^{####} | 0.80 ± 0.79^{###} |

Results: The histopathological diagnosis results showed that under this experimental system, the colon tissue structure of the normal control group animals was clear and layered obviously, and no obvious histological abnormal changes were found. However, animals in the model control group showed significant histological changes of colitis, with the pathological changes being particularly pronounced in the middle segment of the colon. Therefore, pathological diagnosis and analysis were performed and focused on the middle segment of the colon. The pathological changes were mainly manifested as significant thickening of the colonic wall, with the formation of slit-like ulcer lesions on the mucosal surface of the colonic wall. Typical granulomas were observed in the submucosa and muscularis, accompanied by inflammatory cell infiltration predominantly consisting of lymphocytes and plasma cells. Endarteritis of small blood vessels, fibroblast activation, thickening of the small arterial walls, and narrowing of the vascular lumen were observed. Additionally, there was obvious connective tissue proliferation and severe fibrosis process in the submucosa and muscularis, with fibers arranged in bundles. Other features included destruction of crypt structures and hemorrhage surrounding the ulcer lesions. The average histopathological scores for tissue ulcers and inflammatory responses were 2.00 ± 0.00 points. The positive control drug, sulfasalazine, demonstrated a significant inhibitory effect on the progression of colonic ulcers and inflammatory responses in the model animals under the established treatment regimen. Compared to the model control group, the animals in the corresponding treatment group showed a significant reduction in the severity of colonic tissue ulcers and inflammation. The average pathological scores were 0.50 ± 0.53 points and 0.60 ± 0.52 points, respectively, with the improvement showing statistically significant differences (P < 0.05). At the same time, the animals in the positive drug treatment group exhibited significant improvements and healing in the proliferation of connective tissue and fibrosis process in the submucosa and muscularis of the colon.

Test compound I exhibited a significant inhibitory effect on the process of colonic ulcers and inflammatory responses in model animals under the established treatment regimen. The average scores of the animals for colonic tissue ulcers in each treatment group with different doses were 0.80 ± 0.63 points, 0.70 ± 0.67 points, and 0.70 ± 0.67 points, respectively. The average scores for colonic inflammatory response were 1.00 ± 0.67 points, 0.70 ± 0.67 points, and 0.80 ± 0.79 points, respectively. The improvements in all treatment groups showed statistically significant differences compared to the model control group in the same period (P < 0.05). At the same time, in all test compound I treatment groups with different doses, tissue healing and a reduction in the degree of fibrosis were observed in the colon. The pathological scoring results of colon tissue ulcer and inflammatory response of animals in each experimental group are shown in FIG. 2A and FIG. 2B. Representative images of colonic tissue ulcer lesions from animals of each experimental group are shown in FIG. 3.

### 3) Colon tissue fibrosis level

**Table 7. CVF% and HYP content levels in colon tissue of animals in each experimental group**

| Group | CVF (%) of colon middle segment | HYP (µg/g) of colon middle segment |
|---|---|---|
| Normal control group | 0.74 ± 0.32 | 1141.78 ± 106.42 |
| Model control group | 6.39 ± 3.16^{****} | 2109.99 ± 385.85^{****} |
| Positive drug treatment group | 2.67 ± 1.06^{****##} | 1299.79 ± 161.27^{*####} |
| Compound I low dose group | 2.97 ± 1.78^{**##} | 1333.43 ± 155.33^{**####} |
| Compound I medium dose group | 2.36 ± 1.47^{**##} | 1228.40 ± 235.44^{####+} |
| Compound I high dose group | 2.37 ± 1.33^{**##} | 1270.56 ± 287.01^{####} |

Results: Test compound I and the positive control drug sulfasalazine showed a significant inhibitory effect on the process of colon fibrosis in the model animals under the established treatment regimen, which was mainly manifested by inhibiting the proliferation of submucosal fibrous connective tissue and improving the fibrosis of muscularis ulcer lesions. The analysis results of the collagen volume fraction (CVF%, fibrotic area/total area of the colon submucosa and muscularis) of the submucosa and muscularis of the colon middle segment showed that under the experimental conditions, the CVF% in the colon of the normal control group animals was 0.74 ± 0.32%, while the CVF% in the colon of the model control group animals significantly increased to 6.39 ± 3.16% under the same detection and analysis conditions, with a statistically significant difference (P < 0.05) compared to the normal control group. Test compound I and the positive control drug, sulfasalazine, showed a significant inhibitory effect on the increase in CVF% in the colon of model animals under the established treatment regimen. Under the same analysis conditions, their average CVF% values were 2.97 ± 1.78%, 2.36 ± 1.47%, 2.37 ± 1.33%, and 2.67 ± 1.06%, respectively, with a statistically significant difference (P < 0.05) compared to the model control group.

Consistent with the colon CVF% results, the HYP detection results for the colon middle segment tissue showed that, compared to the normal control group (1141.78 ± 106.42 µg/g), the HYP content in the colon of the model control group animals (2109.99 ± 385.85 µg/g) was significantly increased, with a statistically significant difference (P < 0.05) compared to the normal control group. Under the established treatment regimen, the positive control drug sulfasalazine significantly improved the degree of colon fibrosis in model animals. Under the same detection conditions, the HYP content in the colon (1299.79 ± 161.27 µg/g) showed a significant decrease response, with a statistically significant difference (P < 0.05) compared to the model control group.

Under the established treatment regimen, the test compound I simultaneously exhibited a significant therapeutic effect on the process of colon fibrosis in model animals. Compared to the model control group animal, the HYP levels in the colon tissues of the treatment group animals showed a significant decrease response, with values of 1333.43 ± 155.33 µg/g, 1228.40 ± 235.44 µg/g, and 1270.56 ± 287.01 µg/g, respectively. These values showed a statistically significant difference (P < 0.05) compared to the HYP content levels in the corresponding colon segments of the model control group animals. Moreover, its improvement effect exhibited a certain dose-dependent relationship, being effective at a dose of 3.0 mg/kg. The colon tissue fibrosis levels of animals in each experimental group are shown in FIGs. 4A and 4B.

Conclusion: In this experiment, TNBS was used to perform enema intervention on male SD rats, once/2 weeks, for two consecutive rounds, to induce colonic lesions, and establish a rat model of chronic colitis with intestinal fibrosis that is consistent with clinical onset characteristics. The treatment of the test compound I (3.0, 6.0, and 15.0 mg/kg, twice a day) and the positive control drug sulfasalazine (50.0 mg/kg, twice a day) began on the day of model induction. During the treatment period (a continuous 4 weeks), indicators such as changes in body weight of experimental animals, DAI score of colitis disease activity, histopathological detection of colon tissue, and hydroxyproline detection in colon tissue, were monitored to evaluate the therapeutic effect of the test compound I on TNBS-induced SD rats of chronic colitis with intestinal fibrosis. In this experiment, a rat model of chronic colitis with intestinal fibrosis in line with clinical onset characteristics was established. The efficacy evaluation results showed that the compound I had significant therapeutic effect.

### Example 2: In vivo pharmacodynamic study of compound on carbon tetrachloride-induced rat models of liver fibrosis

1. Experimental objective
To evaluate the therapeutic effect of the test compound I on carbon tetrachloride-induced SD rat models of liver fibrosis.
2. Experimental methods
1) Animals: SD rats, specific pathogen-free (SPF) grade, male, aged 7 to 8 weeks at the time of grouping, with 10 mice per group. The animals were supplied by Beijing HFK Bioscience Co., Ltd.
2) Reagents, positive drugs, and test drugs
A. Reagents

**Table 9. Sources of reagents**

| Name | Supplier |
|---|---|
| Carbon tetrachloride | Sigma-Aldrich |
| Hydroxyproline detection kit | Nanjing Jiancheng Bioengineering Institute |
| Corn oil | Shanghai Aladdin Bio-Chem Technology Co., Ltd. |

B. Positive drug: Pirfenidone, supplied by Biochempartner.
C. Test drug: compound I, supplied by Guangzhou JOYO Pharmatech Co., Ltd.
3) Modeling method
Intraperitoneal injection method of carbon tetrachloride: After the adaptation period inspection and observation were qualified, experimental animals were screened by body weight and randomly divided into a normal control group and a model induction group. Starting on the first day of grouping, animals in the model induction group were intraperitoneally injected with a 20% carbon tetrachloride solution dissolved in corn oil, at a modeling frequency of twice a week for six consecutive weeks. After two weeks of carbon tetrachloride induction, animals in the model induction group were randomly subdivided a second time into 5 experimental groups based on their alanine transaminase (ALT) levels (with body weight used as a calibration factor), including the model control group, positive drug treatment group, test compound low dose treatment group, test compound medium dose treatment group, and test compound high dose treatment group. Animals in the normal control group were administered the corresponding vehicle. The experimental period lasted for 6 weeks, during which all animals were given normal access to water and food.
4) Grouping and administration info

**Table 10. Grouping and administration**

| Group | Number of animals | Drug | Route of administration | Dose | Concentration (mg/mL) | Frequency and period of administration |
|---|---|---|---|---|---|---|
| Normal control group | 10 | Test compound vehicle^{a} | Oral gavage | - | - | Twice a day Four consecutive weeks |
| Model control group | 10 | Test compound vehicle^{a} | Oral gavage | - | - | |
| Positive drug treatment group | 10 | Pirfenidone b | Oral gavage | 150 mg/kg | 15 | |
| Test compound low dose treatment group | 10 | Compound I^{b} | Oral gavage | 3 mg/kg | 0.3 | |
| Test compound medium dose treatment group | 10 | Compound I^{b} | Oral gavage | 10 mg/kg | 1 | |
| Test compound high dose treatment group | 10 | Compound I^{b} | Oral gavage | 30 mg/kg | 3 | |

| | | | | | | |
|---|---|---|---|---|---|---|
| Note: a: The test compound vehicle was an aqueous solution of 0.5% (w/v) methylcellulose and 0.2% (v/v) Tween 80. b: An aqueous solution of 0.5% (w/v) methylcellulose and 0.2% (v/v) Tween 80 was used as the vehicle and prepared to the required concentration. | | | | | | |

5) Endpoint sample collection, histopathological analysis, and hydroxyproline content analysis.
A. After euthanizing the animals with pentobarbital sodium anesthesia, the liver was excised and rinsed with normal saline. The liver was divided into three segments: the large lobe, the left lobe, and the right-middle lobe. The large lobe tissue was quickly frozen in liquid nitrogen and stored in a -80°C refrigerator for measuring the total hydroxyproline levels of animal liver samples in each group. The tissues of left lobe and right-middle lobe of liver were fixed in 10% formaldehyde, embedded in paraffin, and sectioned into 3 µm-thick slices. These slices were then deparaffinized in xylene, rehydrated through a graded ethanol series, subjected to Sirius Red staining, dehydrated, cleared, sealed, and observed under a microscope to capture histopathological changes. Routine tissue freezing was performed.
B. Analysis of fibrosis levels in liver tissue sections;

**Table 11. Scoring criteria for liver fibrosis**

| Pathological features | Fibrosis stage | Score |
|---|---|---|
| Normal liver, no fibrosis | S0 | 0 |
| Fibrous proliferation in some portal areas, with or without short fibrous septa | S1 | 1 |
| Fibrous proliferation in most portal areas, with or without short fibrous septa | S1 | 2 |
| Fibrous proliferation in most portal areas, with occasional fibrous bridging between portal areas | S2 | 3 |
| Fibrous proliferation in the portal areas, with obvious fibrous bridging (between portal areas and portal canals, and between portal areas and central veins) | S3 | 5 |
| Obvious fibrous bridging (portal canals to portal canals and/or portal canals to central veins), with occasional fibrous nodules (incomplete cirrhosis, with visible pseudolobular structure) | S3 | 5 |
| Possible or confirmed cirrhosis | S4 | 6 |

Based on Sirius Red staining results of liver sections, 1) fibrosis staging was scored according to the scoring criteria listed in Table 3; 2) using Image J, the liver fibrotic area and the total sample area of animals in each experimental group were counted to calculate the collagen volume fraction (CVF%) for each sample.

### C. Detection of hydroxyproline levels in liver

The liver large lobe samples were processed according to the instructions using the hydroxyproline detection kit. The total hydroxyproline levels in the liver samples of animals in each group were detected.

### 4. Experimental results

1) Liver tissue pathological diagnosis results: The results of Sirius red staining and diagnosis of animal liver in each experimental group during the experimental period are shown in FIG. 5.
   Results: Under this experimental system, the liver of model animals showed obvious liver fibrosis lesions induced by carbon tetrachloride, including lobular structure disorder, obvious collagen proliferation at the interface of liver plate, thickening of connective tissue in the portal area and central vein area, and formation of pseudolobular structure. Under the use of test compound I and the established treatment regimen, it was observed under a light microscope at 200× and 400× magnification that the pseudolobular structure of the liver in some animals in the medium and high dose treatment groups tended to disintegrate, which was mainly manifested in two aspects: narrowing of the fibrous bundles at the liver fibrous bridge sites and the reduction in the number of pseudolobules. At the same time, in each treatment group of the test compound, the degree of hepatocyte fat degeneration and swelling degeneration in the central vein area of the liver was reduced to some extent compared to the model control group, with the medium and high dose being the most obvious. However, the positive control drug pirfenidone treatment group showed no significant improvement.
2) Liver tissue fibrosis level

**Table 12. Liver fibrosis process scores of animals in each experimental group**

| Group | Fibrosis score (points) | CVF (%) | HYP content (µg/g) |
|---|---|---|---|
| Normal control group | 0 | 1.24 ± 0.23 | 307.30 ± 78.13 |
| Model control group | 4.90 ± 0.88 | 8.75 ± 2.39^{****} | 702.84 ± 173.44^{****} |
| Positive drug treatment group | 5.40 ± 0.52 | 6.58 ± 2.20^{****#} | 611.19 ± 183.66^{****} |
| Compound I low dose group | 4.40 ± 0.70 | 5.02 ± 1.42^{****###} | 716.17 ± 105.01^{****} |
| Compound I medium dose group | 4.00 ± 0.94# | 4.87 ± 1.68^{****###} | 560.60 ± 114.04^{****#} |
| Compound I high dose group | 4.20 ± 0.92 | 5.60 ± 1.92^{****##} | 607.79 ± 112.74^{****} |

Results: The degree of liver fibrosis in the model control group animals was obvious, with fibrosis staging all at S3. The average fibrosis score was 4.90 ± 0.88 points. In most of the animals, fibrous bridging was observed between portal canals and portal canals and/or between portal canals and central vein areas of the liver, forming pseudolobular structures. A few animals exhibited clear signs of cirrhosis. Under the established treatment regimen, test compound I demonstrated a certain degree of inhibitory effect on the liver fibrosis process in the model animals. The average liver fibrosis scores of animals in the low, medium, and high dose groups were 4.40 ± 0.70 points, 4.00 ± 0.94 points, and 4.20 ± 0.92 points, respectively. Among these, the medium dose group showed the most significant improvement in fibrosis degree, with a statistically significant difference compared to the model control group (P < 0.05). However, the positive control drug pirfenidone did not show a notable effect on the liver fibrosis process in the model animals, with an average fibrosis score of 5.40 ± 0.52 points, which was not significantly different from the model control group (P > 0.05).

The calculation results of the collagen volume fraction (CVF%) of the animal liver in each experimental group showed that, compared to the normal control group (1.24 ± 0.23%), the CVF% of the animal liver in the model control group was significantly increased, reaching 8.75 ± 2.39%. Under the established treatment regimen, test compound I demonstrated a significant inhibitory effect on the liver fibrosis process in the model animals, with CVF% of 5.02 ± 1.42%, 4.87 ± 1.68%, and 5.60 ± 1.92%, respectively. All of these were significantly different from the model control group (P < 0.05). The positive control drug Pirfenidone showed a significant inhibitory effect on the liver fibrosis degree in the model animals, with a liver CVF% of 6.58 ± 2.20%, which was significantly different from the model control group (P < 0.05).

The detection results of hydroxyproline in the liver tissue of animals in each experimental group showed that the HYP content level in the liver of the model control group animals was 702.84 ± 173.44 µg/g, significantly higher than that of the normal control group animals (307.30 ± 78.13 µg/g), showing a statistically significant difference (P < 0.05). The middle and high dose groups of test compound I and the positive control drug pirfenidone had a certain inhibitory effect on the increase of liver hydroxyproline level of model animals under the established treatment regimen, and the liver HYP levels showed a decreasing trend, which were 560.60 ± 114.04 µg/g, 607.79 ± 112.74 µg/g, and 611.19 ± 183.66 µg/g, respectively, among which the improvement effect of test compound I was the most obvious. There was a significant difference compared to the model control group (P < 0.05). The liver tissue fibrosis levels of animals in each experimental group are shown in FIG. 6.

Conclusion: In this experiment, the liver lesions of male SD rats were induced by intraperitoneal injection of 20% carbon tetrachloride corn oil solution (twice a week for 6 consecutive weeks), and a rat liver fibrosis model was established in line with the clinical onset characteristics. After 2 weeks of continuous induction, the rats were treated with test compound I (3.0, 10.0, and 30.0 mg/kg, twice a day) and the positive control drug pirfenidone (150.0 mg/kg, twice a day) for 4 consecutive weeks. The liver pathological fibrosis scoring results showed that test compound I treatment for 4 weeks could significantly inhibit the process of liver fibrosis in the model animals. The average liver fibrosis scores of animals for the low, medium, and high dose groups were 4.40 ± 0.70 points, 4.00 ± 0.94 points, and 4.20 ± 0.92 points, respectively, which were significantly lower than that of the model control group (4.90 ± 0.88 points). The quantitative analysis results of collagen volume fraction (CVF%) in liver sections showed that the CVF% levels for the low, medium, and high dose groups of test compound I and the positive control drug pirfenidone were 5.02 ± 1.42%, 4.87 ± 1.68%, 5.60 ± 1.92%, and 6.58 ± 2.20%, respectively. All these values were significantly lower than that of the model control group (8.75 ± 2.39%, P < 0.05), indicating that all treatment groups exhibited a significant inhibitory effect on liver fibrosis. Consistent with the liver pathological fibrosis analysis results, the hydroxyproline levels in the liver tissue of the medium dose group of test compound I were also significantly lower compared to the model control group (560.60 ± 114.04 µg/g vs 702.84 ± 173.44 µg/g, P < 0.05). The efficacy evaluation results showed that the compound I had significant therapeutic effect.

Although the foregoing describes specific embodiments of the present disclosure, it should be understood by those skilled in the art that these are only examples. Various changes or modifications can be made to these embodiments without departing from the principles and essence of the present disclosure. Therefore, the scope of protection for the present disclosure is defined by the appended claims.

## Claims

1. A use of substance A in the manufacture of a medicament, wherein the substance A is a compound of formula I or a pharmaceutically acceptable salt thereof; the medicament is a medicament for treating and/or preventing intestinal fibrosis or non-alcoholic liver fibrosis;

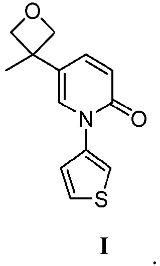

2. The use according to claim 1, wherein the use meets one or more of the following conditions:
(1) the intestinal fibrosis is intestinal fibrosis caused by inflammatory bowel disease, preferably intestinal fibrosis in Crohn's disease;
(2) the medicament comprises the substance A and a pharmaceutically acceptable carrier;
(3) the medicament is administered via the gastrointestinal tract, such as orally;
(4) the medicament is administered at a frequency of once a day, twice a day, or three times a day, preferably twice a day;
(5) the dosage form of the medicament is a solid formulation;
(6) the single dose of the medicament is the mass ratio of the substance A to a subject/patient, wherein the mass ratio is 3 to 40 mg/kg, preferably 3 mg/kg, 6 mg/kg, 10 mg/kg, 15 mg/kg, 30 mg/kg, or 40 mg/kg;
(7) the daily dose of the medicament is the mass ratio of the substance A to a subject/patient, wherein the mass ratio is 3 to 80 mg/kg, preferably 3 mg/kg, 6 mg/kg, 12 mg/kg, 15 mg/kg, 30 mg/kg, 40 mg/kg, 60 mg/kg, or 80 mg/kg;
and (8) the unit dose of the substance A is 25 to 900 mg, preferably 50 to 600 mg, more preferably 200 to 400 mg.

3. The use according to claim 1, wherein the use meets one or more of the following conditions:
(1) the compound of formula I is present in the medicament at a concentration of 0.3 to 3 mg/mL, preferably 0.3 mg/mL, 0.6 mg/mL, 1 mg/mL, 1.5 mg/mL, or 3 mg/mL;
(2) when the medicament is a medicament for treating and/or preventing intestinal fibrosis, the single dose of the medicament is the mass ratio of the substance A to a subject/patient, wherein the mass ratio is 3 to 40 mg/kg, preferably 3 mg/kg, 6 mg/kg, 10 mg/kg, 15 mg/kg, 30 mg/kg, or 40 mg/kg;
(3) when the medicament is a medicament for treating and/or preventing non-alcoholic liver fibrosis, the single dose of the medicament is the mass ratio of the substance A to a subject/patient, wherein the mass ratio is 3 to 40 mg/kg, preferably 3 mg/kg, 6 mg/kg, 10 mg/kg, 15 mg/kg, 30 mg/kg, or 40 mg/kg;
(4) when the medicament is manufactured to treat and/or prevent intestinal fibrosis, the daily dose of the medicament is the mass ratio of the substance A to a subject/patient, wherein the mass ratio is 3 to 80 mg/kg, preferably 3 mg/kg, 6 mg/kg, 12 mg/kg, 15 mg/kg, 30 mg/kg, 40 mg/kg, 60 mg/kg, or 80 mg/kg;
and (5) when the medicament is manufactured to treat and/or prevent non-alcoholic liver fibrosis, the daily dose of the medicament is the mass ratio of the substance A to a subject/patient; the mass ratio is 3 to 80 mg/kg, preferably 3 mg/kg, 6 mg/kg, 12 mg/kg, 15 mg/kg, 30 mg/kg, 40 mg/kg, 60 mg/kg, or 80 mg/kg.

4. The use according to claim 1, wherein the mass fraction of the compound of formula I in the medicament is 3% to 50%, preferably 10% to 20%.

5. A pharmaceutical composition, wherein the pharmaceutical composition comprises substance A and a pharmaceutically acceptable carrier, and the substance A is a compound of formula I or a pharmaceutically acceptable salt thereof; 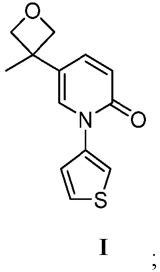 in the pharmaceutical composition, the compound of formula I has a mass fraction of 3% to 50%.

6. The pharmaceutical composition according to claim 5, wherein in the pharmaceutical composition, the compound of formula I has a mass fraction of 10% to 20%.

7. A use of the pharmaceutical composition according to claim 5 or 6 in the manufacture of a medicament for treating and/or preventing intestinal fibrosis or non-alcoholic liver fibrosis;
preferably,
the intestinal fibrosis is intestinal fibrosis caused by inflammatory bowel disease, preferably intestinal fibrosis in Crohn's disease.

8. A method for treating and/or preventing intestinal fibrosis or non-alcoholic liver fibrosis, wherein the method comprises administering a therapeutically effective amount of substance A or a medicament to a subject/patient;
the medicament comprises the substance A and a pharmaceutically acceptable carrier; the substance A is a compound of formula I or a pharmaceutically acceptable salt thereof;

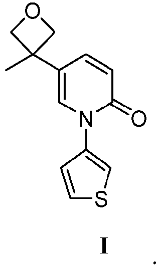

9. The method according to claim 8, wherein the method meets one or more of the following conditions:
(1) the intestinal fibrosis is intestinal fibrosis caused by inflammatory bowel disease, preferably intestinal fibrosis in Crohn's disease;
(2) the medicament comprises the substance A and a pharmaceutically acceptable carrier;
(3) the medicament is administered via the gastrointestinal tract, such as orally;
(4) the medicament is administered at a frequency of once a day, twice a day, or three times a day, preferably twice a day;
(5) the dosage form of the medicament is a solid formulation;
(6) the single dose of the medicament is the mass ratio of the substance A to a subject/patient, wherein the mass ratio is 3 to 40 mg/kg, preferably 3 mg/kg, 6 mg/kg, 10 mg/kg, 15 mg/kg, 30 mg/kg, or 30 mg/kg;
(7) the daily dose of the medicament is the mass ratio of the substance A to a subject/patient, wherein the mass ratio is 3 to 80 mg/kg, preferably 3 mg/kg, 6 mg/kg, 12 mg/kg, 15 mg/kg, 30 mg/kg, 40 mg/kg, 60 mg/kg, or 80 mg/kg;
and (8) the unit dose of the substance A is 25 to 900 mg, preferably 50 to 600 mg, more preferably 200 to 400 mg.

10. The method according to claim 8, wherein the method meets one or more of the following conditions:
(1) the compound of formula I is present in the medicament at a concentration of 0.3 to 3 mg/mL, preferably 0.3 mg/mL, 0.6 mg/mL, 1 mg/mL, 1.5 mg/mL, or 3 mg/mL;
(2) when the medicament is a medicament for treating and/or preventing intestinal fibrosis, the single dose of the medicament is the mass ratio of the substance A to a subject/patient, wherein the mass ratio is 3 to 40 mg/kg, preferably 3 mg/kg, 6 mg/kg, 10 mg/kg, 15 mg/kg, 30 mg/kg, or 40 mg/kg;
(3) when the medicament is a medicament for treating and/or preventing non-alcoholic liver fibrosis, the single dose of the medicament is the mass ratio of the substance A to a subject/patient, wherein the mass ratio is 3 to 40 mg/kg, preferably 3 mg/kg, 6 mg/kg, 10 mg/kg, 15 mg/kg, 30 mg/kg, or 40 mg/kg;
(4) when the medicament is manufactured to treat and/or prevent intestinal fibrosis, the daily dose of the medicament is the mass ratio of the substance A to a subject/patient, wherein the mass ratio is 3 to 80 mg/kg, preferably 3 mg/kg, 6 mg/kg, 12 mg/kg, 15 mg/kg, 30 mg/kg, 40 mg/kg, 60 mg/kg, or 80 mg/kg;
and (5) when the medicament is manufactured to treat and/or prevent non-alcoholic liver fibrosis, the daily dose of the medicament is the mass ratio of the substance A to a subject/patient; the mass ratio is 3 to 80 mg/kg, preferably 3 mg/kg, 6 mg/kg, 12 mg/kg, 15 mg/kg, 30 mg/kg, 40 mg/kg, 60 mg/kg, or 80 mg/kg.
